# EUROPEAN PATENT APPLICATION

(11) **EP 2 778 996 A1**
(43) Date of publication of application: **17.09.2014**
(21) Application number: 14155916.1
(22) Date of filing: 20.02.2014
(51) Int. Cl.: G06F 19/00

(54) **Methods and systems for determining patient and geographic specific medical reports**

(30) Priority: 15.03.2013 US 201313834837
(71) Applicant: IDEXX LABORATORIES, INC., Westbrook, Maine 04092 (US)
(72) Inventor: Brazell, Robert B., Cape Elizabeth, ME Maine 04107 (US); Ayers, Jonathan W., Portland, ME Maine 04102 (US); Basford, Eric Christopher, Scarborough, ME Maine 04074 (US); Durgin, Matthew B., Scarborough, ME Maine 04074 (US); Stamaris, John, Gorham, ME Maine 04038 (US)
(74) Representative: HOFFMANN EITLE

(57) **Abstract**

Methods and systems for determining patient and geographic specific medical reports are provided. In one example, a method includes receiving a result of a diagnostic test performed on a patient, and creating, by a computing device, an output of the result in a report. The method further includes based on the result being indicative of a condition, providing by the computing device in the report a geographic representation of one or more incidences of the condition reported in a geographic region associated with the patient. In other example methods, based on the result being indicative of the absence of at least one tick-borne disease or heartworm disease, a geographic representation of one or more incidences of the disease reported in a geographic region associated with the patient may be provided in the report.

## Description

### BACKGROUND

Unless otherwise indicated herein, the materials described in this section are not prior art to the claims in this application and are not admitted to be prior art by inclusion in this section.

In the field of veterinary medicine, there are debates on an ideal visit frequency for animals to visit their veterinarian for a checkup. For adult animals, a once yearly visit is often recommended. As an animal reaches an older age, more frequent visits may be recommended, such as every six months. Health problems associated with old age are common, and can advance rapidly. A quick check every six months may be beneficial to improve chances of identifying any disease at an early stage to increase chances for successful treatment.

In addition, some animals may require an annual booster vaccine, which may give rise to a once-yearly health check. As animal vaccines have improved in recent years, in some instances, full annual vaccination may not be needed. Rather, vaccine recommendations may be offered on an individual basis, such that one animal might continue to need a once yearly injection (e.g., if the animal lives in an area where there are higher risks) and another animal might only need a vaccine every three years. When yearly vaccines are not required, a once-yearly health check may be less likely to occur.

Of course, frequent health checks cost money, and have to be fit into a budget. Animal owners may want to be able to justify the need for the health check, and veterinarians continue to develop ways to urge animal owners to maintain recommended visit frequencies.

### SUMMARY

In one example, a method is provided that comprises receiving a result of a diagnostic test performed on a patient, and creating, by a computing device, an output of the result in a report. The method also comprises based on the result being indicative of a condition, providing by the computing device in the report a geographic representation of one or more incidences of the condition reported in a geographic region associated with the patient.

In another example, a computer readable storage medium having stored therein instructions, that when executed by a computing device, cause the computing device to perform functions. The functions may comprise receiving a result of a diagnostic test performed on a patient, and creating an output of the result in a report. The functions may also comprise based on the result being indicative of a condition, providing in the report a geographic representation of one or more incidences of the condition reported in a geographic region associated with the patient.

In still another example, a system is provided that comprises at least one processor, and data storage comprising program instructions executable by the at least one processor to cause the at least one processor to perform functions. The functions may comprise receiving a result of a diagnostic test performed on a patient, and creating an output of the result in a report. The functions may also comprise based on the result being indicative of a condition, providing in the report a geographic representation of one or more incidences of the condition reported in a geographic region associated with the patient.

In yet another example, another method is provided that comprises receiving a result of at least one diagnostic test performed on a patient. The at least one diagnostic test may be selected from a group of tests that detect a presence or absence of a tick-borne disease and/or heartworm disease. The method also comprises creating, by a computing device, an output of the result in a report, and based on the result being indicative of the absence of at least one tick-borne disease or heartworm disease, providing by the computing device in the report a geographic representation of one or more incidences of the disease reported in a geographic region associated with the patient.

These as well as other aspects, advantages, and alternatives, will become apparent to those of ordinary skill in the art by reading the following detailed description, with reference where appropriate to the accompanying figures.

### BRIEF DESCRIPTION OF THE FIGURES

Figure 1 illustrates an example system for processing medical data.
Figure 2 is a block diagram of an example method to create and provide personalized medical reports, in accordance with at least some embodiments described herein.
Figure 3 is an example of a report that may be created based on performing diagnostic tests on a patient.
Figure 4 is another example of a report that may be created based on performing diagnostic tests on a patient.
Figure 5 is a block diagram of another example method to create and provide personalized medical reports, in accordance with at least some embodiments described herein.
Figure 6 is a block diagram of yet another example method to create and provide personalized medical reports, in accordance with at least some embodiments described herein.

### DETAILED DESCRIPTION

The following detailed description describes various features and functions of the disclosed systems and methods with reference to the accompanying figures. In the figures, similar symbols identify similar components, unless context dictates otherwise. The illustrative system and method embodiments described herein are not meant to be limiting. It may be readily understood that certain aspects of the disclosed systems and methods can be arranged and combined in a wide variety of different configurations, all of which are contemplated herein.

Within examples, methods and systems for determining patient and geographic specific medical reports are provided. In one example, a method includes receiving a result of a diagnostic test performed on a patient, and creating, by a computing device, an output of the result in a report. The method further includes based on the result being indicative of a condition, providing by the computing device in the report a geographic representation of one or more incidences of the condition reported in a geographic region associated with the patient. In other example methods, based on the result being indicative of the absence of at least one tick-borne disease or heartworm disease, a geographic representation of one or more incidences of the disease reported in a geographic region associated with the patient may be provided in the report.

In some examples, veterinarians utilize reports to explain benefits for the testing as well as to provide recommendations (when needed) for additional testing. Veterinarians may want to help the pet owner understand the test results, rather than just providing the results themselves (which may be unintelligible to non-veterinarians). Using examples herein, client-friendly and patient-specific reports are configured to place information into context of the pet for the client. Medical or computing devices may be configured to receive and process diagnostic test results in specific manners per pet to provide an informative analysis of the results.

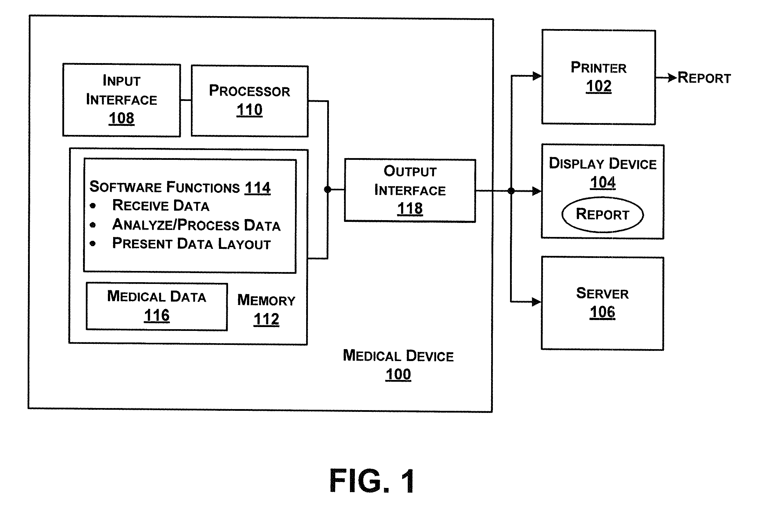

Referring now to the figures, Figure 1 illustrates an example system for processing medical data. The system includes a medical device 100 that outputs to a printer 102, a display device 104 (e.g., a computer monitor, tablet, or smart phone), a server 106, or other computing devices (not shown).

The medical device 100 includes an input interface 108 that receives medical readings from a patient, such as a person or animal, or that receives medical data from other medical devices or stored medical data from a server, for example. In addition, the input interface 108 may include any standard medical interface for collecting desired medical readings from a patient, either human or animal.

The input interface 108 may be coupled to a processor 110 to provide the received data to the processor 110. The input interface 108 may also receive inputs from a user including instructions for processing the medical readings. The processor 110 accesses memory 112 to execute any of software functions 114 stored therein, such as to receive the medical readings, analyze and process the readings, and to present the processed or other generated data to the printer 102, the display device 104, or server 106, for example. The processor 110 may further access the memory 112 to retrieve other stored medical data 116, or past medical results, and may combine the medical data 116 with the received medical readings to be output through an output interface 118. The output interface 118 may allow the medical device 100 to communicate with other devices. In some examples, the output interface 118 may also maintain and manage records of data received and sent by the medical device 100.

Further, the input interface 108 and the output interface 118 may be any standard computer interface and may include, for example, a keyboard. However, other interfaces may be used as well. In addition, the input interface 108 and the output interface 118 may be configured as wired (e.g., wired serial bus such as a universal serial bus or a parallel bus) or wireless connections (e.g., Bluetooth® radio technology, communication protocols described in IEEE 802.11 (including any IEEE 802.11 revisions), or Cellular technology (such as GSM, CDMA, UMTS, EV-DO, WiMAX, or LTE) among other possibilities.

A system bus or an equivalent system may also be provided to enable communications between various elements of the medical device 100, the printer 102, the display device 104, or the server 106.

The processor 110 may operate according to an operating system, which may be any suitable commercially available embedded or disk-based operating system, or any proprietary operating system. The processor 110 may comprise one or more smaller central processing units, including, for example, a programmable digital signal processing engine. The processor 110 may also be implemented as a single application specific integrated circuit (ASIC) to improve speed and to economize space.

The memory 112 may include main memory and secondary storage. The main memory may include random access memory (RAM). Main memory can also include any additional or alternative memory device or memory circuitry. Secondary storage can be provided as well and may be persistent long term storage, such as read only memory (ROM), optical or magnetic disks, compact-disc read only memory (CD-ROM), or any other volatile or non-volatile storage systems. The memory 112 may include more software functions than illustrated as well, for example, executable by the processor 110 to record or receive signals from a patient, either human or animal, and interpret the signals as medical readings. The software functions 114 may be provided using machine language instructions or software with object-oriented instructions, such as the Java programming language. However, other programming languages (such as the C++ programming language for instance) could be used as well.

It will be apparent to those of ordinary skill in the art that the methods described herein may be embodied in a computer program product that includes one or more computer readable media, as described as being present within the medical device 100. For example, a computer readable medium can include a readable memory device, such as a hard drive device, a CD-ROM, a DVD-ROM, or a computer diskette, having computer readable program code segments stored thereon. The computer readable medium can also include a communications or transmission medium, such as, a bus or a communication link, either optical, wired or wireless having program code segments carried thereon as digital or analog data signals.

It should be further understood that this and other arrangements described herein are for purposes of example only. As such, those skilled in the art will appreciate that other arrangements and other elements (e.g. machines, interfaces, functions, orders, and groupings of functions, etc.) can be used instead, and some elements may be omitted altogether according to the desired results. Further, many of the elements that are described are functional entities that may be implemented as discrete or distributed components or in conjunction with other components, in any suitable combination and location. In some examples, the medical device 100 could include hardware objects developed using integrated circuit development technologies, or yet via some other methods, or the combination of hardware and software objects that could be ordered, parameterized, and connected in a software environment to implement different functions described herein. Also, the hardware objects could communicate using electrical signals, with states of the signals representing different data. It should also be noted that the medical device 100 generally executes application programs resident at the medical device 100 under the control of the operating system of the medical device 100.

The medical device 100 may be of the type provided by IDEXX Laboratories, Inc., of Westbrook, Maine, USA. For example, the medical device 100 may be one of those within the IDEXX VetLab® Suite that delivers information on blood chemistries, proteinuria, electrolytes, hematology, endocrinology and blood gases. Such devices include the VetTest® instrument, the LaserCyte® hematology analyzer, the VetLyte® electrolyte analyzer, the VetStat® blood gas analyzer, or a device for reading an immunoassay device, such as the SNAPshot® Dx instrument or the SNAP® reader, for example.

The medical device 100 may additionally or alternatively take the form of a handheld device, laptop, personal computer, a workstation, or a server. The medical device 100 may include an input device, such as a keyboard and/or a two or three-button mouse, if so desired. One skilled in the art of computer systems will understand that the example embodiments are not limited to any particular class or model of computer employed for the medical device 100 and will be able to select an appropriate system.

The medical device 100 may be configured to output a medical report to the printer 102, the display device 104, and/or the server 106. Alternatively, the output can include a transmission (e.g. fax, e-mail) to a remote location (such as from a reference laboratory to a clinic). The server 106 may be an e-mail server, and can be configured to provide the medical report to identified e-mail accounts.

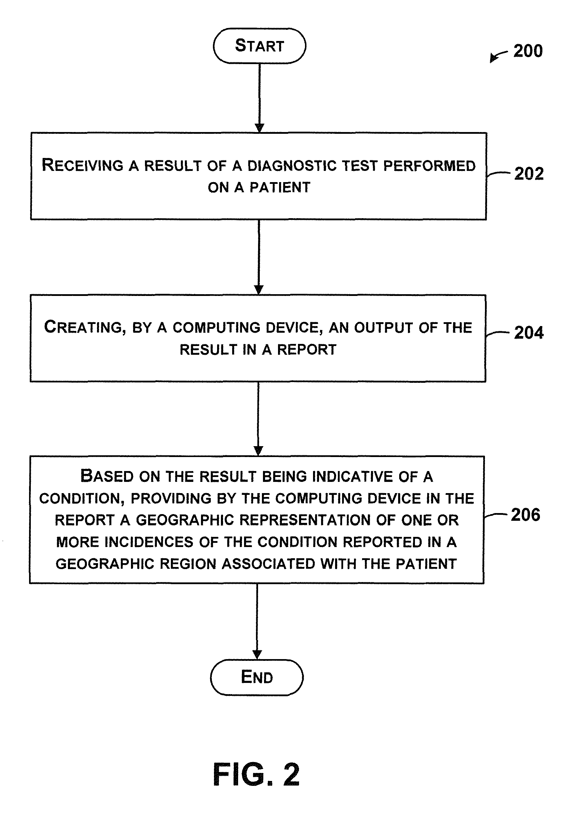

Figure 2 is a block diagram of an example method 200 to create and provide personalized medical reports, in accordance with at least some embodiments described herein. Method 200 shown in Figure 2 presents an embodiment of a method that, for example, could be used or executed by the medical device 100, for example, and may be performed by a device, a server, or a combination of any other computing device components. Method 200 may include one or more operations, functions, or actions as illustrated by one or more of blocks 202-206. Although the blocks are illustrated in a sequential order, these blocks may in some instances be performed in parallel, and/or in a different order than those described herein. Also, the various blocks may be combined into fewer blocks, divided into additional blocks, and/or removed based upon the desired implementation.

In addition, for the method 200 and other processes and methods disclosed herein, the flowchart shows functionality and operation of one possible implementation of present embodiments. In this regard, each block may represent a module, a segment, or a portion of program code, which includes one or more instructions executable by a processor for implementing specific logical functions or steps in the process. The program code may be stored on any type of computer readable medium, for example, such as a storage device including a disk or hard drive. The computer readable medium may include a non-transitory computer readable medium, for example, such as computer-readable media that stores data for short periods of time like register memory, processor cache and Random Access Memory (RAM). The computer readable medium may also include non-transitory media, such as secondary or persistent long term storage, like read only memory (ROM), optical or magnetic disks, compact-disc read only memory (CD-ROM), for example. The computer readable media may also be any other volatile or non-volatile storage systems. The computer readable medium may be considered a computer readable storage medium, a tangible storage device, or other article of manufacture, for example.

In addition, for the method 200 and other processes and methods disclosed herein, each block in Figure 2 may represent circuitry that is wired to perform the specific logical functions in the process.

At block 202, the method 200 includes receiving a result of a diagnostic test performed on a patient. In some examples, the method may be performed by a computing device that performs the diagnostic test on the patient and directly receives or records a reading from the patient. In other examples, samples from the patient may be sent to a lab, and the computing device may receive results of the diagnostic test from the lab. The results of the diagnostic test may be associated with a patient identifier or an electronic medical record (EMR) of the patient, for example.

The diagnostic test may be any test performed on a patient, and may include, for example, tests that detect biomarkers such as antibodies, antigens, DNA and RNA.

At block 204, the method 200 includes creating, by a computing device, an output of the result in a report. The output may take many forms, and may include a summary of the result of the diagnostic test, for example. The report may include a written report provided by a printer, a graphical report provided on a display (e.g., provided by a graphical user interface (GUI) in any type of format), an electronic report (e.g., provided in an e-mail). For example, an email can be provided to an owner of an animal that underwent diagnostic testing, and the report can be provided as an attachment, and further comments can be provided in a message in the email (e.g., example comments may be descriptive of outcomes of the tests: "Good news! We have screened [the patient's] blood for common conditions, and your pet's organ systems appear to be functioning normally.").

At block 206, the method 200 includes based on the result being indicative of a condition, providing by the computing device in the report a geographic representation of one or more incidences of the condition reported in a geographic region associated with the patient. In some examples, the geographic representation includes a geographic map and a representation of a number of reported incidences of the condition per geographic regions of the geographic map. Thus, the computing device may perform a programmatic function of obtaining, generating, and/or providing into the report the geographic representation, and the function is based on the test results having an indication of the condition.

In some examples, the result of the diagnostic test may be incorporated into the number of incidences of the condition being reported in the geographic region associated with the patient, and the geographic representation can be tailored to illustrate or highlight the patient's test result compared to all other reported incidences. For example, the geographic region associated with the patient can be highlighted (e.g., displayed in a certain color, shaded, etc.) to illustrate how many incidences have been reported in the patient's hometown for comparison.

The condition may include any number of conditions that can be detected or diagnosed based on the specific diagnostic test. Example conditions for diagnostic tests performed on animals include tests for lyme disease, an *ehrlichia canis* infection, an *anaplasma phagocytophilum* infection, and a presence of heartworms.

In some examples, determination that the results are indicative of a condition includes comparing the test results with reference values or expected values. However, because each patient is unique, reference values may not actually be in a normal range for a given patient. Thus, test results for a test may be established as a baseline of values for a patient while the patient is or appears healthy. Subsequent test results may be compared against the baseline for the specific patient, rather than or in addition to comparing against reference range data, to determine if any areas have changed in the baseline which can be used as an indication of abnormal results.

Thus, testing may be performed tests to establish baseline results for future comparisons. The test results may be processed to generate the report as a summary of the baseline for the patient.

The function of providing the geographic representation at block 206 may include determining which condition is represented by the test result, accessing a database to retrieve stored reported incidences of the condition, and generating the geographic representation based on the stored reported incidences. In other examples, a database may store the geographic representation, which can be retrieved.

In some examples, the function of providing the geographic representation in the report at block 206 may be based on a number of reported incidences being above a threshold or based on a risk in the geographic regions being above a threshold. For example, if a number of reported incidences are low, the data may not be statistically significant so as to provide meaningful information. Thus, the method 200 may also include first analyzing or processing the geographic representation or data used to generate the geographic representation to make a determination of whether to provide the geographic representation in the report.

In further examples, based on the result being indicative of the condition the method 200 may also include providing in the report a recommendation for another diagnostic test, providing in the report a recommendation for treatment, providing in the report a recommendation of a laboratory to conduct another diagnostic test, and/or providing in the report a notice of a discount for performing another diagnostic test. For example, when results of the diagnostic test are indicative of lyme disease, it may be recommended to perform a subsequent test to determine quantitative C₆ antibody levels in the patient to provide another metric as an indication that the patient has lyme disease. Thus, the report can include a recommendation for performing the quantitative C₆ test. The report can include additional helpful information for the animal owner, for example, including a coupon for a discount to perform additional testing.

In examples, the report can be tailored for the specific patient, the particular diagnostic test, and the particular geography in which the patient resides or is associated. In further instances, a particular geography in which the patient resides may be used to further process test results. For instance, if test results are inconclusive as to whether the patient has lyme disease, and the patient resides in an area with high incidences of reported lyme disease, a patient or patient's owner may be more likely to follow a recommendation for additional testing to resolve the inconclusive test results. The report can be useful to make such decisions.

In some examples, such as when the patient is an animal, the report may include an image of a specific breed of the animal and an indication of an affected area of the animal on the image based on the condition. The report could additionally or alternatively include an image of the specific patient, and an indication of an affected area of the patient on the image based on the condition.

In some examples, additional information can be provided in the report including images of x-rays or other lab information to educate the patient or patient's owner of the type of testing performed.

The report can be further tailored by providing in the report textual comments describing the result of the diagnostic test.

The computing device may further be configured to provide in the report an indication of previous results of the diagnostic test performed on the patient for comparison with current results. Often times, a comparison of current results with historical or baseline results of a patient can be used as a basis for a high confidence diagnosis of a condition, rather than or in addition to a comparison with default or reference range results of typical patients.

In further examples, based on the result being indicative of the condition the method 200 may also include sending the report to a veterinarian or to the patient. For example, the report may be sent in an email to the patient along with comments from the doctor.

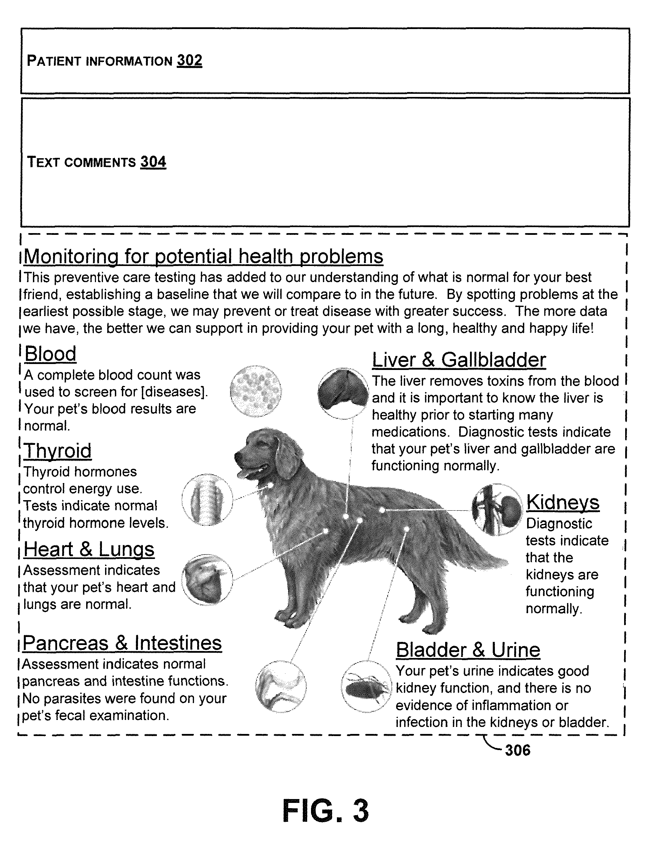

Figure 3 is an example of a report that may be created based on performing diagnostic tests on a patient. The report may include patient information 302, such as any of name, age, year of birth, date tested, type of species, type of report, etc. The report may also include text comments 304 provided by a user (e.g., veterinarian) to describe data of the report. Example comments may include "We have screened [the patient's] blood for common conditions, and your pet's organ system appear to be functioning normally. This preventive care testing is very important for us to sport potential health issues before [the patient] shows symptoms, and to obtain a baseline of blood values while your pet is healthy. Also, [the patient] is free of diseases transmitted by mosquitoes and ticks. Keep up the good work with regular tick checks and by following your pet's prevention plan!"

The report is shown to include baseline data section 306 including details of different tests performed on the patient, information about importance and benefits of testing, as well as a picture of a breed of an animal representative of the patient and call-outs to specific areas on the patient corresponding to the tests. The section 306 may specify benefits of testing (e.g., the preventive care testing establishes a baseline for comparison in the future to track subtle changes more effectively, and improve ability to detect disease even when values are normal, or before any outward signs of illness can be seen). Example tests may be performed for blood counts to screen for anemia, inflammation, infection, stress, leukemia, bleeding problems, hydration, and ability to fight infection. Example tests may also be performed for thyroid functions to determine thyroid hormone levels (e.g., thyroid hormones control how quickly the body uses energy, and thyroid function can become abnormal and cause illness), for heart and lungs to assess normal functioning levels, for pancreas and intestines to assess normal functions or presence of parasites (e.g., the pancreas is a small organ located near the small intestines and is responsible for producing several digestive enzymes and hormones that help regulate metabolism, and the intestines are needed for digestion and absorption of nutrients from food), for liver and gallbladder to assess normal functions (e.g., the liver is a large organ with many different functions and produces bile which is stored in the gallbladder and is important for fat digestion. The liver makes protein and cholesterol and is involved in most aspects of protein, carbohydrate and fat metabolism, and also removes toxins from the blood and it is important to know the liver is healthy prior to starting many medications), for kidneys (e.g., kidneys are responsible for filtering metabolic waste products, excess sodium, and water from the blood stream, as well as conserving vital electrolytes), and bladder and urine testing as well (e.g., a urinalysis can be performed to help assess the health of your pet's kidneys and bladder).

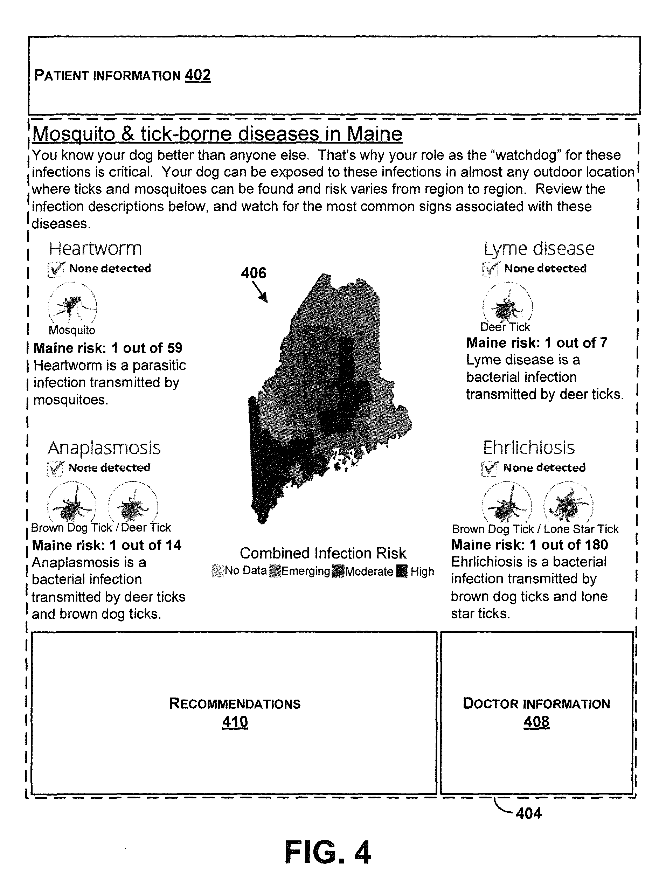

Figure 4 is another example of a report that may be created based on performing diagnostic tests on a patient. The report may again include patient information 402 similar to the report shown in Figure 3. The report may also include a summary section 404 describing a description of benefits of the tests performed (e.g., symptoms may be hard to detect in the early stages so it is recommended to perform this parasitic disease screen at the dog's annual checkup, and to work to find infections early, because early intervention is beneficial for a lifetime of good health).

The summary section 404 can also include details of diseases for which diagnostic tests were performed on the patient, and results of those tests. For example, the report shown in Figure 4 is a detailed report directed to diagnostic testing for mosquito and tick-borne diseases, and includes specific results for testing of heartworm (e.g., heartworm is a parasitic infection transmitted by mosquitoes, and symptoms may include mild persistent cough, fatigue, being tired after moderate exercise, weight loss, and reduced appetite), anaplasmosis (e.g., anaplasmosis is a bacterial infection transmitted by deer ticks and brown dog ticks, and symptoms may include lack of energy, lameness, swollen or painful joints, and loss of appetite), lyme disease (e.g., lyme disease is a bacterial infection transmitted by deer ticks, and symptoms may include lameness, reluctance to move, swollen or painful joints, lack of energy, and urinating more), and ehrlichiosis (e.g., ehrlichiosis is a bacterial infection transmitted by brown dog ticks and lone star ticks, and symptoms may include loss of appetite, depression, lameness, swollen or painful joints, bloody nose, and pale gums).

The report in Figure 4 may provide further details, such as statistics indicating a risk for contracting the disease in a geographic region (e.g., the report illustrates example statistics for the state of Maine). The risks may be illustrated as statistical numbers, such as 1 out of 59 for Heartworm, 1 out of 14 for anaplasmosis, etc. Risks may be calculated as ratios of a number of positive test results per a number of patients tested, or as ratios of a number of positive test results per a population of a given region. The risks are also shown, in this example, as a geographic representation 406 of one or more incidences of the disease or condition reported in the geographic region associated with the patient. The geographic representation 406 in this example shows the state of Maine with shadings representative of various infection risks based on report incidences in regions, counties, or other areas of the state.

The geographic representation 406 may be in other forms as well, such as mapping of areas and indications of specific numbers of reported incidences of diseases per area, for example. The geographic representation may also include a geographic map and a representation of a risk (e.g., various shading of areas on the map) of being exposed to an infection related to the condition per geographic regions of the geographic map.

The report in Figure 4 further includes a section for veterinarian information 408, such as name, address, contact information, etc. The report in Figure 4 also includes a section for recommendations 410. The recommendations 410 may include a recommendation for when a follow-up check-up should occur, advice on how to maintain a healthy lifestyle, advice for performing at-home check-ups, references to other information. The recommendations 410 may further include recommendations for additional testing based on results of the tests performed possibly to validate or further verify the existence or absence of a disease, recommendations for particular labs to perform the tests, or even discounts/coupons for testing, healthcare items, specific food products, or various advertising, for example.

Within examples herein, methods may be executed to generate one report that is a combination of the examples shown in Figures 3-4, or to generate one or the other of the reports shown in Figures 3-4.

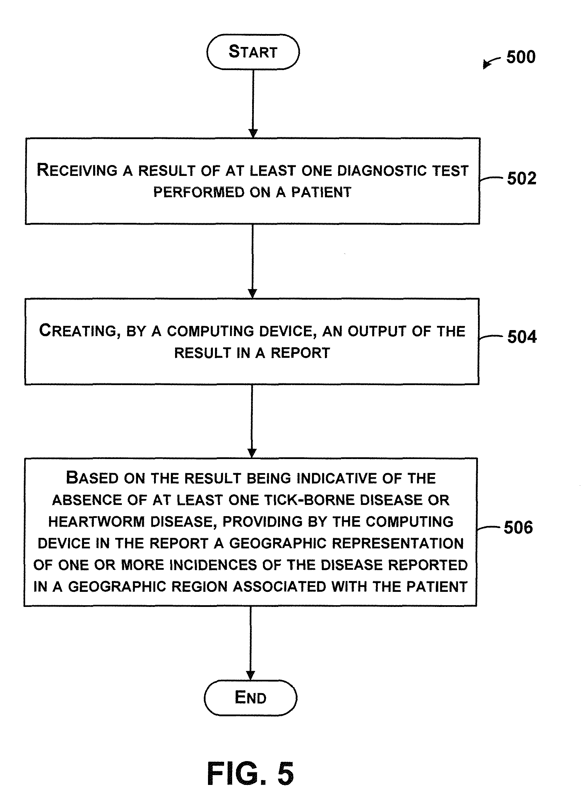

Figure 5 is a block diagram of another example method 500 to create and provide personalized medical reports, in accordance with at least some embodiments described herein. Method 500 shown in Figure 5 presents an embodiment of a method that, for example, could be used or executed by the medical device 100, for example, and may be performed by a device, a server, or a combination of any other computing device components. In addition, each block may represent a module, a segment, or a portion of program code, which includes one or more instructions executable by a processor for implementing specific logical functions or steps in the process.

At block 502, the method 500 includes receiving a result of at least one diagnostic test performed on a patient. Within examples, the diagnostic test may be a limited test that is specifically directed to detecting a presence or absence of a tick-borne disease and/or heartworm disease within animals. Of course, the diagnostic test may be any type of test depending on an application of the method 500.

At block 504, the method 500 includes creating, by a computing device, an output of the result in a report, such as in a format similar to either of the reports shown in Figures 3-4.

At block 506, the method 500 includes based on the result being indicative of the absence of at least one tick-borne disease or heartworm disease, providing by the computing device in the report a geographic representation of one or more incidences of the disease reported in a geographic region associated with the patient. In this example, a report may only provide the geographic representation (e.g., including a geographic map and a representation of a number of reported incidences of the disease per geographic regions of the geographic map) when the test results indicate that the patient does not have the disease. The report can be illustrative of the amount of incidences of the disease, and the fact that the patient does not test positive is a sign of good healthcare and preventive care by the patient or patient's owner. In some instances, when test results are good (e.g., no diseases detected), little information is provided to the patient other than the "good news". However, within examples herein, when test results are good, additional information is provided such as the geographic representation of reported incidences to inform of risks of contracted the disease in the area. The reports can convey the need to follow recommended check-up schedules due to possible high risks in certain geographic regions of contracting specific diseases.

In further examples, based on the result being indicative of the absence of the condition the method 500 may also include sending the report to a doctor or to the patient. For example, the report may be sent in an email to the pet owner along with comments from the veterinarian.

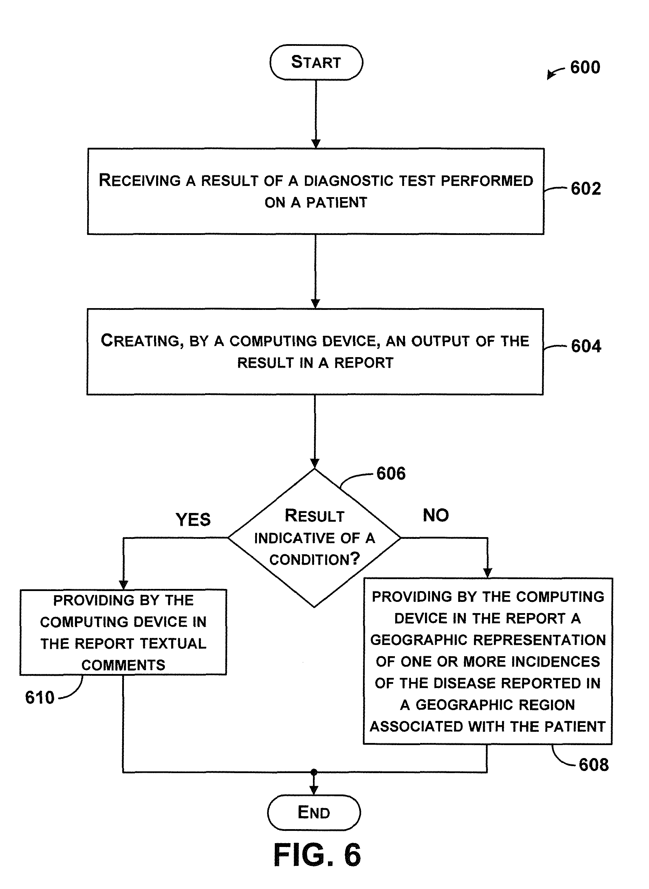

Figure 6 is a block diagram of yet another example method 600 to create and provide personalized medical reports, in accordance with at least some embodiments described herein. Method 600 shown in Figure 6 presents an embodiment of a method that, for example, could be used or executed by the medical device 100, for example, and may be performed by a device, a server, or a combination of any other computing device components. In addition, each block may represent a module, a segment, or a portion of program code, which includes one or more instructions executable by a processor for implementing specific logical functions or steps in the process.

At block 602, the method 600 includes receiving a result of a diagnostic test performed on a patient, and at block 604 the method 600 includes creating by a computing device an output of the result in a report. Blocks 602 and 604 may be similar to previous corresponding functions described in the methods of Figures 2 and 5.

At block 606, the method 600 includes determining if the result is indicative of a condition. In some examples, a determination can be made based on a comparison of the test results with results known to indicate that the condition is present. In examples, the function of receiving the result of the test may also include receiving a qualitative (or quantitative) indication of whether the results indicate presence or absence of the condition. A computing device may then be programed to perform functions based on the indication received.

At block 608, the method includes based on the result not being indicative of the condition, providing by the computing device in the report a geographic representation of one or more incidences of the disease reported in a geographic region associated with the patient. Thus, similar to the method 500 in Figure 5, when the condition is determined to be absent, the geographic representation may be determined and provided in the report.

At block 610, the method includes based on the result being indicative of the condition, providing by the computing device in the report textual comments. In some examples, the textual comments may provide details or an explanation of the detected condition, or may indicate for the patient or the patient's owner to contact the doctor to discuss the test results. In some examples, indeterminate or possibly bad test results may not be proper to be provided in the report.

Thus, within examples, the report can be customized to provide details when results are indicative of the condition. When test results for a disease are positive, the test results can be conveyed in a manner to provide helpful advice and recommendations for a course of treatment. The geographic representation of reported incidences may be disregarded when test results are positive, according to the method 600. In this example, the report can focus on positive aspects of the test results or recommendations for moving forward, rather than providing the geographic report of incidences when the patient tests positive as well.

In still further examples, example methods (such as the methods in Figures 2, 5, and 6) may include additional functions such as determining a recommendation for a next check-up based on the test results and/or based on the number of reported incidences of conditions in the geographic region associated with the patient. For instance, although a patient may test negative for a condition (e.g., lyme disease) and the normal recommendation for a following check-up is about one-year, if the patient lives in an area deemed a high-risk lyme disease area (based on the geographic representation of incidences of lyme disease reported), the computing device may determine a recommendation for a following check-up to be sooner than one-year. Thus, the computing device may utilize a number of reported incidences or whether the patient resides in high risk areas, as indicated in the geographic representation, as a basis for determining certain recommendations. The computing device may programmatically perform such calculations, such as reducing default follow-up timeframes by half, for example, when the number of reported incidences is above a threshold or when the patient resides in or near high risk areas, for example.

Any of the example methods described herein may be performed by a medical device (as shown in Figure 1), or any computing device, or portions of functions of the methods may be performed by a server that is in communication with such devices. As an example implementation, a computing device may receive test results of a patient from a server, device, or other testing equipment at a lab, and then may access another server or database to retrieve the reported incidences or geographic representation for the report (based on whether the results indicate the presence or absence of a condition and based on settings of the computing device to perform functions of method 200 in Figure 2, method 500 in Figure 5, or method 600 in Figure 6). The computing device may generate the report, and can be programmed to send or provide the report to the veterinarian or to the pet owner, and the sending of the report to the pet owner can be based on whether the test results are deemed positive or negative.

It should be understood that arrangements described herein are for purposes of example only. As such, those skilled in the art will appreciate that other arrangements and other elements (e.g. machines, interfaces, functions, orders, and groupings of functions, etc.) can be used instead, and some elements may be omitted altogether according to the desired results. Further, many of the elements that are described are functional entities that may be implemented as discrete or distributed components or in conjunction with other components, in any suitable combination and location.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the claims, along with the full scope of equivalents to which such claims are entitled. It is also to be understood that the terminology used herein is for the purpose of describing particular embodiments only, and is not intended to be limiting.

The invention also relates to the following embodiments, numbered (i) to (x):
Embodiment (i). The computer readable storage medium as defined in claim 14, wherein the condition includes conditions selected from the group consisting of lyme disease, *ehrlichia canis* infection, *anaplasma phagocytophilum* infection, and the presence of heartworms.
Embodiment (ii). The computer readable storage medium as defined in claim 14 or embodiment (i), wherein the geographic representation includes a geographic map and a representation of a number of reported incidences of the condition per geographic regions of the geographic map.
Embodiment (iii). The computer readable storage medium as defined in claim 14 or embodiment (i) or (ii), wherein the functions further comprise based on the result being indicative of the condition, providing in the report a recommendation selected from the group consisting of a recommendation for another diagnostic test, a recommendation for treatment, and a recommendation of a laboratory to conduct another diagnostic test.
Embodiment (iv). The computer readable storage medium as defined in claim 14 or any one of embodiments (i) to (iii), wherein the functions further comprise based on the result being indicative of lyme disease, providing in the report a recommendation for performing a test to determine quantitative C6 antibody levels in the patient.
Embodiment (v). The computer readable storage medium as defined in claim 14 or any one of embodiments (i) to (iv), wherein the functions further comprise providing by the computing device in the report one or more textual comments describing the result of the diagnostic test.
Embodiment (vi). The system as defined in claim 15, wherein the geographic representation includes a geographic map and a representation of a number of reported incidences of the condition per geographic regions of the geographic map.
Embodiment (vii). The system as defined in claim 15 or embodiment (vi), wherein the functions further comprise:
   providing in the report an image of the patient; and
   providing an indication of an affected area of the patient on the image based on the condition.
Embodiment (viii). A method comprising:
   receiving a result of at least one diagnostic test performed on a patient, the at least one diagnostic test selected from a group of tests that detect a presence or absence of a tick-borne disease and/or heartworm disease;
   creating, by a computing device, an output of the result in a report; and
   based on the result being indicative of the absence of at least one tick-borne disease or heartworm disease, providing by the computing device in the report a geographic representation of one or more incidences of the disease reported in a geographic region associated with the patient.
Embodiment (ix). The method of embodiment (viii), further comprising providing by the computing device in the report one or more textual comments describing the result of the at least one diagnostic test.
Embodiment (x). The method of embodiment (viii) or (ix), wherein the geographic representation includes a geographic map and a representation of a number of reported incidences of the disease per geographic regions of the geographic map.

## Claims

1. A method comprising:
receiving a result of a diagnostic test performed on a patient;
creating, by a computing device, an output of the result in a report; and
based on the result being indicative of a condition, providing by the computing device in the report a geographic representation of one or more incidences of the condition reported in a geographic region associated with the patient.

2. The method of claim 1, wherein the condition includes conditions selected from the group consisting of lyme disease, *ehrlichia canis* infection, *anaplasma phagocytophilum* infection, and the presence of heartworms.

3. The method of claim 1 or 2, wherein the diagnostic test includes tests that detect biomarkers selected from the group consisting of antibodies, antigens, DNA and RNA.

4. The method of claim 1 or 2, wherein the diagnostic test includes tests that detect a presence or absence of at least one of a tick-borne disease and heartworm disease, and the condition includes the absence of at least one tick-borne disease or heartworm disease.

5. The method according to any one of the preceding claims, wherein the geographic representation includes a geographic map and a representation of a number of reported incidences of the condition per geographic regions of the geographic map.

6. The method according to any one of claims 1 to 4, wherein the geographic representation includes a geographic map and a representation of a risk of being exposed to an infection related to the condition per geographic regions of the geographic map.

7. The method according to any one of the preceding claims, further comprising based on the result being indicative of the condition, providing in the report at least one of:
a recommendation for another diagnostic test;
a recommendation for treatment;
a recommendation of a laboratory to conduct another diagnostic test; and
a notice of a discount for performing another diagnostic test.

8. The method according to any one of the preceding claims, further comprising:
determining a recommendation for a follow-up check-up based on a number of the one or more incidences of the condition reported in the geographic region associated with the patient or based on whether the patient resides in a high risk areas as indicated in the geographic representation; and
providing the recommendation in the report.

9. The method according to any one of the preceding claims, further comprising providing in the report data indicating a risk of contracting the condition in the geographic region associated with the patient.

10. The method according to any one of the preceding claims, wherein the patient is an animal, and the method further comprises:
providing in the report an image of a specific breed of the animal; and
providing an indication of an affected area of the animal on the image based on the condition.

11. The method according to any one of the preceding claims, further comprising based on the result being indicative of lyme disease, providing in the report a recommendation for performing a test to determine quantitative C₆ antibody levels in the patient.

12. The method according to any one of the preceding claims, further comprising providing by the computing device in the report an indication of previous results of the diagnostic test performed on the patient.

13. The method according to any one of the preceding claims, further comprising providing by the computing device in the report one or more textual comments describing the result of the diagnostic test.

14. A computer readable storage medium having stored therein instructions, that when executed by a computing device, cause the computing device to perform functions comprising:
receiving a result of a diagnostic test performed on a patient;
creating an output of the result in a report; and
based on the result being indicative of a condition, providing in the report a geographic representation of one or more incidences of the condition reported in a geographic region associated with the patient.

15. A system, comprising:
at least one processor; and
data storage comprising program instructions executable by the at least one processor to cause the at least one processor to perform functions comprising:
receiving a result of a diagnostic test performed on a patient;
creating an output of the result in a report; and
based on the result being indicative of a condition, providing in the report a geographic representation of one or more incidences of the condition reported in a geographic region associated with the patient.
